# EUROPÄISCHE PATENTSCHRIFT

(11) **EP 3 685 861 B1**
(45) Veröffentlichungstag und Bekanntmachung des Hinweises auf die Patenterteilung: **02.12.2026**
(21) Anmeldenummer: 20153101.9
(22) Anmeldetag: 22.01.2020
(51) Int. Cl.: A61L 2/24, A61L 2/26, B67D 7/02

(54) **SAUGLANZE FÜR DIE ENTNAHME VON FLÜSSIGKEIT AUS EINEM BEHÄLTER**
SUCTION LANCE FOR REMOVING LIQUID FROM A CONTAINER
LANCE D'ASPIRATION DESTINÉE À L'ENLÈVEMENT DE LIQUIDE D'UN RÉCIPIENT

(30) Priorität: 28.01.2019 DE 102019102033
(43) Veröffentlichungstag der Anmeldung: 29.07.2020
(73) Patentinhaber: Miele & Cie. KG, 33332 Gütersloh (DE)
(72) Erfinder: Leifeld, Ludger, 59227 Ahlen (DE); Dyck, Heinrich, 33719 Bielefeld (DE); Jostes, Dirk, 33602 Bielefeld (DE)

(56) Entgegenhaltungen:
- EP-A1- 2 868 622
- WO-A1-2011/100937
- WO-A1-2016/137940
- WO-A2-2005/108280
- WO-A2-2008/008392
- DE-U1- 9 115 677

## Beschreibung

Die Offenbarung betrifft eine Sauglanze für die Entnahme von Flüssigkeit aus einem Behälter, insbesondere für die Entnahme von Prozesschemikalien aus einem Kanister eines Reinigungs- und/oder Desinfektionsgerät, mit einem Saugrohr und einem Koppelelement, wobei das Koppelelement der lösbaren Anordnung es Saugrohrs an einem behälterseitigen Anschlussstutzen dient.

Reinigungs- und/oder Desinfektionsgeräte im Sinne der Erfindung sind insbesondere programmgesteuerte Waschmaschinen sowie programmgesteuerte Reinigungs- und/oder Desinfektionsgeräte, die als solche aus dem Stand der Technik an sich bekannt sind, weshalb es eines gesonderten druckschriftlichen Nachweises an dieser Stelle nicht bedarf. Programmgesteuerte Reinigungs- und/oder Desinfektionsgeräte dienen der Reinigung und/oder Desinfektion von zu reinigenden und/oder zu desinfizierenden Gütern und verfügen zu diesem Zweck über einen Behandlungsraum, der die zu reinigenden und/oder zu desinfizierenden Güter im bestimmungsgemäßen Verwendungsfall aufnimmt. Typische Reinigungs- und/oder Desinfektionsgeräte sind insbesondere Spülmaschinen für den Haushalt oder den gewerblichen oder medizinischen Bereich, insbesondere Haushaltsgeschirrspüler, Laborspüler, Gastronomiespüler, Reinigungsautomaten für medizinische Geräte und/oder dergleichen Einrichtungen.

Zur Erzielung eines optimierten Reinigungs-, Desinfektions- und/oder Trocknungsergebnisses finden typischerweise flüssige Prozesschemikalien Verwendung, die im Zuge eines Reinigungs-, Desinfektions- und/oder Trocknungsvorgangs dem Behandlungsraum des Reinigungs- und/oder Desinfektionsgeräts zugeführt werden. Bei solchen Prozesschemikalien handelt es sich beispielsweise um Reinigungs- und/oder Desinfektionschemikalien, die je nach Programmsteuerung zu unterschiedlichen Zeitpunkten eingesetzt und dem Behandlungsraum des Reinigungs- und/oder Desinfektionsgeräts dosiert zugeführt werden.

Zum Zwecke der Dosierung flüssiger Prozesschemikalien sind aus dem Stand der Technik Dosiervorrichtungen bekannt, die unter anderem über eine Sauglanze verfügen. Im bestimmungsgemäßen Verwendungsfall reicht die Sauglanze in einen Vorratsbehälter hinein und taucht in die von diesem Vorratsbehälter bevorratete Prozesschemikalie ein.

Die Dosiervorrichtung verfügt des Weiteren über eine an die Sauglanze strömungstechnisch angeschlossene Dosierpumpe. Bei einem Betrieb der Dosierpumpe wird die Prozesschemikalie dem Vorratsbehälter über die Sauglanze entnommen und gelangt mittels einer daran strömungstechnisch angeschlossenen Zuführungsleitung in den Behandlungsraum des Reinigungs- und/oder Desinfektionsgeräts.

Als Vorratsbehälter für die Prozesschemikalien kommen typischerweise Kanister mit einem Kanistervolumen von mehreren Litern zum Einsatz. Ist die von einem solchen Kanister bevorratete Menge an Prozesschemikalien verbraucht, ist der leere Kanister gegen einen neuen, das heißt einen mit der Prozesschemikalien befüllten Kanister auszuwechseln. Zu diesem Zweck ist die Sauglanze dem leeren Kanister zu entnehmen und in den neuen, noch mit Prozesschemikalien befüllten Kanister einzuführen.

Zur Lagesicherung einer in einen Vorratsbehälter hineinragenden Sauglanze dient nach dem Stand der Technik ein Deckel, der korrespondierend zu dem Anschlussstutzen des Vorratsbehälters ausgebildet ist, durch den hindurch die Sauglanze in den vom Vorratsbehälter bereitgestellten Innenraum hineinragt. Der Anschlussstutzen des Vorratsbehälters verfügt typischerweise über ein Außengewinde, mit dem der Deckel der Sauglanze im bestimmungsgemäßen Verwendungsfall verschraubt ist, zu welchem Zweck der Deckel an die geometrische Ausgestaltung des Anschlussstutzens angepasst ausgebildet ist und ein korrespondierend zum Außengewindes des Anschlussstutzens ausgebildetes Innengewinde aufweist.

Die geometrische Ausgestaltung sowohl der Vorratsbehälter, als auch der davon bereitgestellten Anschlussstutzen ist nicht durchgehend normiert und vom jeweiligen Hersteller des Vorratsbehälters abhängig. Es sind deshalb seitens des Herstellers eines Reinigungs- und/oder Desinfektionsgeräts eine Vielzahl von nach Größe und Form unterschiedlich ausgebildeten Deckeln bereitzustellen, so dass dann verwenderseitig der zu einem bestimmten Vorratsbehälter beziehungsweise dessen Anschlussstutzen passende Deckel auszusuchen und mit der Sauglanze zwecks Anordnung am Vorratsbehälter zu kombinieren ist. Dies wird sowohl herstellerseitig als auch verwenderseitig als nachteilig empfunden.

Herstellerseitig ist das Mitliefern einer Vielzahl von unterschiedlich ausgebildeten Deckeln sowohl aufwendig, als auch kostenintensiv. Dabei ist aufgrund der Vielzahl von unterschiedlichen Anschlussgeometrien nicht einmal sichergestellt, dass einer der herstellerseitig mitgelieferten Deckel zum verwenderseitig eingesetzten Vorratsbehälter kompatibel ist.

Verwenderseitig wird es insbesondere als nachteilig empfunden, dass die Auswahl des passenden Deckels nicht immer einfach ist, da es verwenderseitig zum Teil nur sehr schwer festzustellen ist, welche Geometrien im Einzelnen vorliegen, beispielsweise hinsichtlich der Gewindeausgestaltung. Dies kann verwenderseitig bei der Montage zu erheblichen Problemen und Aufwendungen führen.

Um dem vorstehenden Problem zu begegnen, sind aus dem Stand der Technik einfache Kanisterabdeckungen bekannt geworden, die den Anschlussstutzen des Vorratsbehälters lediglich oberseitig abdecken. Ein tatsächlich dichter Abschluss des vorratsbehälterseitigen Anschlussstutzens wird dadurch aber nicht erreicht, so dass bei einem Umkippen des Vorratsbehälters Prozesschemikalie in großer Menge aus dem Vorratsbehälter austreten kann, was unerwünscht ist. Insofern haben sich einfache Kanisterabdeckungen nicht bewährt.

Gemäß einem weiteren Stand der Technik können sogenannte Reduzierstücke zum Einsatz kommen, die im Bedarfsfall als Zwischenglied zwischen Deckel und Anschlussstutzen dienen. Das Kompatibilitätsproblem ist aber auch mit solchen Reduzierstücken tatsächlich nicht beseitigt. Zudem sitzen solche Reduzierstücke nicht fest am Anschlussstutzen und können sich Lösen und dabei große Mengen an Prozesschemikalie austreten lassen. Durch die Verwendung von Reduzierstücken erhöht sich auch die Anzahl der Dichtstellen und damit einhergehend die potentielle Gefahr von Undichtigkeiten. Auch dies gilt es zu vermeiden, weshalb sich Reduzierstücke nicht bewährt haben.

Die EP 2 868 622 A1 offenbart eine Sauglanze für die Entnahme von Prozesschemikalien aus einem Kanister eines Reinigungs- und/oder Desinfektionsgeräts, wobei die Sauglanze ein Saugrohr und ein konusförmiges Koppelelement zum Verschließen des Anschlussstutzen des Kanisters aufweist.

Die WO 2008/008392 A2 und die WO 2005/108280 A2 offenbaren jeweils eine Kopplungseinrichtung, welche zur Entnahme einer Flüssigkeit aus einem Behälter dient.

Die WO 2016/137940 A1 offenbart einen Closed-Loop-Konnektor, welcher ebenfalls zur Entnahme einer Flüssigkeit aus einem Behälter dient. Es ist ausgehend vom Vorbeschrieben die **Aufgabe** der Erfindung, eine Sauglanze mit einem Saugrohr und einem Koppelelement vorzuschlagen, die bei einer gleichzeitig dichten Anordnung an einem Anschlussstutzen eines Vorratsbehälters konstruktiv eine vereinfachte Handhabung durch den Verwender gestattet.

Zur **Lösung** dieser Aufgabe wird mit der Erfindung eine Sauglanze mit den Merkmalen von Anspruch 1 vorgeschlagen.

Das Koppelelement ist in Abkehr zum Stand der Technik nicht als starrer Deckel ausgebildet. Es wird mit der Erfindung vielmehr ein größenvariables Koppelelement vorgeschlagen, das eine sichere und verwenderseitig schnell auszubildende Anordnung beziehungsweise Befestigung der Sauglanze am Anschlussstutzen des Vorratsbehälters gestattet. Das Koppelelement ist zu diesem Zweck erfindungsgemäß größen- und/oder formkompatibel ausgebildet, und zwar zu einer Mehrzahl von der in der Größe und/oder Form unterschiedlichen Anschlussstutzen.

Von Vorteil der erfindungsgemäßen Ausgestaltung ist, dass ein und dasselbe Koppelelement mit einer Mehrzahl von unterschiedlich ausgebildeten Anschlussstutzen gleichermaßen bestimmungsgemäß verwendbar ist. Dies reduziert die herstellerseitig bereitzustellende Anzahl an Koppelelementen. Zudem ergibt sich eine verwenderseitige Vereinfachung in der Handhabung, da im Unterschied zum Stand der Technik eine verringerte Anzahl an Koppelelementen zur Anordnung der Sauglanze am Vorratsbehälter zur Wahl steht. Mit dem erfindungsgemäßen Koppelelement kann mithin ein Spektrum an unterschiedlich ausgebildeten Anschlussstutzen gleichermaßen kombiniert werden, so dass es im Unterschied zum Stand der Technik nicht weiter erforderlich ist, je Anschlussstutzen einen speziell hierauf angepassten Deckel vorzusehen.

Gemäß einem weiteren Merkmal der Offenbarung ist vorgesehen, dass das Koppelelement zumindest abschnittsweise aus einem elastisch verformbaren Material gebildet ist. Demnach ist die Größen- und Formvariabilität des Koppelelements dadurch erreicht, dass dieses zumindest abschnittsweise aus einem elastisch verformbaren Material gebildet ist, was es gestattet, das Koppelelement auf unterschiedlich ausgebildete Anschlussstutzen aufsetzen zu können. Aufgrund der elastischen Verformbarkeit ist das Koppelelement zumindest abschnittsweise federelastisch ausgebildet, so dass es verwenderseitig zur Anpassung an einen behälterseitigen Anschlussstutzen größen- und/oder formgedehnt werden kann. Nach einem verwenderseitigen Aufsetzen des Koppelelements auf den Anschlussstutzen zieht sich dieses federelastisch bedingt zusammen und legt sich hierdurch klemmend, insbesondere weitgehend flüssigkeitsdicht, an den Anschlussstutzen an. Bedingt durch die Gewindeform am Anschlussstutzen ist dabei keine absolute Dichtheit erreicht, dies ist aber auch nicht erforderlich bzw. gewünscht, da während der Medienentnahme das gleiche Luftvolumen nachströmen muss. Bei einem Umkippen des Vorratsbehälters ist die eventuell austretende Menge an Prozesschemikalie aufgrund der Ausbildung des Koppelelements dennoch marginal.

Das elastisch verformbare Material ist gemäß einem weiteren Merkmal der Offenbarung Silikon, Gummi und/oder dergleichen. Je nach eingesetztem Material deckt ein und dasselbe Koppelelement eine Vielzahl von in der Größe und/oder der Form unterschiedlich ausgebildeten Anschlussstutzen ab und kann mit diesen gleichsam bestimmungsgemäß Verwendung finden. Da mit ein und demselben Koppelelement eine Vielzahl von unterschiedlich ausgebildeten Anschlussstutzen zusammenwirkt, ist es ausreichend, zur zumindest weitestgehenden Abdeckung sämtlicher Anschlussformen und -größen, das heißt -geometrien nur einige wenige Koppelelemente vorzusehen, beispielsweise drei oder vier Koppelelemente. Diese sind seitens beispielsweise des Herstellers des Reinigungs- und/oder Desinfektionsgeräts für eine verwenderseitige Benutzung bereitzustellen, womit dann über ein insgesamt breites Anwendungsspektrum Kompatibilität zu marktüblichen Kanister- beziehungsweise Anschlussstutzenausgestaltungen gegeben ist.

Gemäß einem weiteren Merkmal der Offenbarung ist vorgesehen, dass das Koppelelement nach Art einer Kappe ausgebildet ist und einen Kappendeckel sowie eine umlaufende Wandung aufweist, wobei die Wandung den Anschlussstutzen im endmontierten Zustand umgreifend ausgebildet ist. Das Koppelelement ist demgemäß kappenähnlich ausgestaltet. Es ist im bestimmungsgemäßen Verwendungsfall über den Anschlussstutzen des Vorratsbehälters gestülpt, wobei ein Kappendeckel vorgesehen ist, der die anschlussstutzenseitige Öffnung verschließt und wobei eine umlaufende Wandung vorgesehen ist, die den Anschlussstutzen außenseitig umgreift, jeweils bezogen auf den endmontierten Zustand des erfindungsgemäßen Koppelelements. Durch diese Ausgestaltung ist im endmontierten Zustand eine sichere und lagegenaue Positionierung des Koppelelements und damit auch der Sauglanze in Relation zum Anschlussstutzen gegeben. Das wandungsseitige Umgreifen des Anschlussstutzens stellt zudem eine weitgehend flüssigkeitsdichte, insbesondere ausreichend flüssigkeitsdichte Anordnung des Koppelelements am Anschlussstutzen sicher, so dass auch im Falle eines Umkippens des Vorratsbehälters ein leckagebedingtes Auslaufen von Prozesschemikalie weitestgehend unterbunden ist.

Es ist gemäß einem weiteren Merkmal der Offenbarung vorgesehen, dass die Wandung auf ihrer dem Anschlussstutzen im endmontierten Zustand zugewandten Innenoberfläche mit einer Haltekontur ausgerüstet ist. Bei einer solchen Haltekontur kann es sich beispielsweise um Vorsprünge, Rastnasen oder -nuten, ein Gewinde und/oder dergleichen Einrichtung handeln. Dabei besteht der Sinn und Zweck einer solchen Haltekontur darin, eine innige und dichte Verbindung zwischen Koppelelement und Anschlussstutzen auszubilden. Diese Ausgestaltung erweist sich insbesondere in Kombination mit dem Merkmal des elastischen verformbaren Materials als vorteilhaft, da sich hierdurch im bestimmungsgemäßen Verwendungsfall ein Eindrücken der koppelelementseitigen Haltekontur in ein vom Anschlussstutzen bereitgestelltes Außengewinde ergibt. Dabei ist es für ein dichtes Anliegen des Koppelelements am Anschlussstutzen nicht erforderlich, dass die Haltekontur in korrespondierender Entsprechung zum Außengewinde des Anschlussstutzens ausgebildet ist. Die elastische Verformbarkeit des Koppelelementmaterials sorgt dafür, dass sich die Haltekontur in die vom Anschlussstutzen außenumfangsseitig bereitgestellte Gegenkontur eindrückt, so dass es sowohl zu einer kraftschlüssigen als auch zu einer formschlüssigen oder weitgehend formschlüssigen Verbindung zwischen Koppelelement und Anschlussstutzen kommt.

Gemäß einem weiteren Merkmal der Offenbarung ist vorgesehen, dass der Kappendeckel eine Durchtrittsöffnung für das Saugrohr aufweist. Im endmontierten Zustand durchragt das Saugrohr der Sauglanze diese vom Kappendeckel bereitgestellte Durchtrittsöffnung.

Gemäß einer ersten Ausführungsform der Offenbarung ist vorgesehen, dass der Kappendeckel im endmontierten Zustand flüssigkeitsdicht am Saugrohr anliegt. Gemäß dieser Ausführungsform ist die Durchtrittsöffnung im Kappendeckel auf den Durchmesser des Saugrohres abgestimmt, so dass ein flüssigkeitsdichtes Anliegen des Kappendeckels am Saugrohr im endmontierten Zustand sichergestellt ist. In diesem Fall erfolgt eine Entlüftung über die nicht absolut dichtende Verbindung zwischen Kappe und der Außenkontur des Anschlussstutzens.

Gemäß dieser ersten Alternative ist gemäß einem weiteren Merkmal der Offenbarung vorgesehen, dass der Kappendeckel eine Lüftungsöffnung aufweist. Aufgrund des flüssigkeitsdichten Anliegens des Kappendeckels am Saugrohr ist es für ein Nachströmen von Luft im Saugfall erforderlich, eine entsprechende Lüftungsöffnung vorzusehen. Diese ist erfindungsgemäß vom Kappendeckel bereitgestellt.

Gemäß einer zweiten Alternative ist keine direkte Anordnung des Koppelelements am Saugrohr vorgesehen, sondern eine indirekte, und dies unter Zwischenordnung eines Schlittens zur längsverschieblichen Anordnung des Koppelelements am Saugrohr. Bei dem Schlitten kann es sich bevorzugter Weise um eine Hülse handeln, die in Längsrichtung des Saugrohres verschiebbar am Saugrohr angeordnet ist. Die Hülse steht wiederum ihrerseits mit dem Koppelelement in Wirkverbindung, so dass im Ergebnis das Koppelelement in Längsrichtung des Saugrohrs verschiebbar am Saugrohr angeordnet ist, und zwar unter Zwischenordnung der als Schlitten dienenden Hülse.

Zur Anordnung des Koppelelements an der Hülse ist gemäß einem weiteren Merkmal vorgesehen, dass die Hülse auf ihrer dem Saugrohr im endmontierten Zustand abgewandten Außenoberfläche eine Befestigungskontur aufweist. Diese Befestigungskontur dient der vorzugsweise lösbaren Anordnung des Koppelelements an der Hülse. In einfacher Weise kann so verwenderseitig die Hülse, auch Hülsenelement genannt, mit in ihrer geometrischen Ausgestaltung jeweils unterschiedlich ausgebildeten Koppelelementen wahlweise bestückt werden.

Dabei handelt es sich bei der Befestigungskontur bevorzugter Weise um eine hülsenaußenseitig umlaufende Rille. Im endmontierten Zustand kommt das Koppelelement in dieser Rille randseitig zu liegen.

Die zweite Alternative mit der längsverschieblichen Hülse erbringt den Vorteil, dass das Koppelelement dem Grunde nach verschleißfrei in Längsrichtung des Saugrohrs in Relation zu diesem verschoben werden kann. Denn die Zwischenordnung des Hülsenelements bedingt, dass ein ungewollter Abrieb des Koppelelements vermieden ist, wie dieser bei einer direkten Anordnung des Koppelelements am Saugrohr auftreten kann.

Die nach der Offenbarung vorgesehene zweite Ausführungsform sieht einen beispielsweise als Hülsenelement ausgebildeten Grundträger vor, der verschiebbar auf dem Saugrohr angebracht ist. Dieser Grundträger kann je nach Kanistergröße auf die richtige Höhe eingestellt werden. Auf einen solchen verschiebbaren Grundträger kann gemäß erster Ausführungsalternative aber auch verzichtet werden, wenn das Koppelelement eine passende Durchtrittsöffnung aufweist, um direkt auf dem Saugrohr montiert zu werden. In diesem Fall benötigt das Koppelelement ein eigenes Lüftungsloch, um Luft in den Kanister während eines Saugvorgangs einströmen lassen zu können. Der Grundträger verfügt über eine Aussparung, die als Belüftungsöffnung dient, womit ein Nachströmen von Luft in den Vorratsbehälter während eines Saugvorgangs ermöglicht ist. Darüber hinaus kann auch Luft zwischen Saugrohr und Grundträger nachströmen. Der Grundträger besitzt ferner einen Aufnahmebereich, der das elastische, beispielsweise gummielastische Koppelelement aufnehmen kann.

Das Koppelelement dient dank seiner elastischen Ausgestaltung der Abdichtung und Verrastung zwischen Sauglanze und Kanister und lässt sich verwenderseitig einfach aufschieben, wobei es sich selber am Grundträger hält. Beim Einführen der Sauglanze in den Kanister stülpt sich die Wandung, das heißt der Bund des Koppelelements über das Gewinde des kanisterseitigen Anschlussstutzens. Das Koppelelement wird dabei gedehnt und hat somit eine genügend große Haftkraft, um nicht abzufallen.

Durch optional vorgesehene Haltekonturen im Inneren des Koppelelements verkrallt sich dieses zusätzlich mit dem kanisterseitigen Anschlussgewinde. Damit ist auch im Kanisterumfall verhindert, dass die Sauglanze relativ zum Kanister wegkippt. Durch die elastische Eigenschaft des Koppelelements dichtet sie über das Gewinde und der oberen planaren Fläche des Anschlussstutzens ab. Diese Verkrall- und Dichtfunktion ist über einen großen Durchmesserbereich an Kanisteranschlussstutzen und unabhängig vom vorhandenen Anschlussstutzengewinde, das heißt insbesondere der Gewindesteigung gegeben.

Mit der Erfindung wird des Weiteren vorgeschlagen ein Reinigungs- und/oder Desinfektionsgerät, mit der Sauglanze des Anspruchs 1. Ein Reinigungs- und/oder Desinfektionsgerät, das mit einer erfindungsgemäßen Sauglanze ausgerüstet ist, erbringt die schon vorstehend erläuterten Vorteile.

Weitere Merkmale und Vorteile der Erfindung ergeben sich aus der nachfolgenden Beschreibung anhand der Figuren. Dabei zeigen:
- Fig. 1: in schematisch perspektivischer Darstellung eine Sauglanze;
- Fig. 2: in einer teilgeschnittenen Seitenansicht die Sauglanze im endmontierten Zustand;
- Fig. 3: in geschnittener Seitenansicht ausschnittsweise das Koppelelement der Sauglanze gemäß Schnittlinie B-B nach Fig. 2 gemäß einer ersten Ausführungsform;
- Fig. 4: das Koppelelement nach Fig. 3 gemäß einer zweiten Ausführungsform;
- Fig. 5: das Koppelelement nach Fig. 3 gemäß einer dritten Ausführungsform;
- Fig. 6: in schematisch perspektivischer Darstellung einen mit einer Sauglanze ausgerüsteten Kanister während einer bestimmungsgemäßen Montage;
- Fig. 7: in schematisch perspektivischer Darstellung eine weitere Montagesituation und
- Fig. 8: in schematisch perspektivischer Darstellung ein mit einer Sauglanze ausgerüstetes Reinigungs- und/oder Desinfektionsgerät.

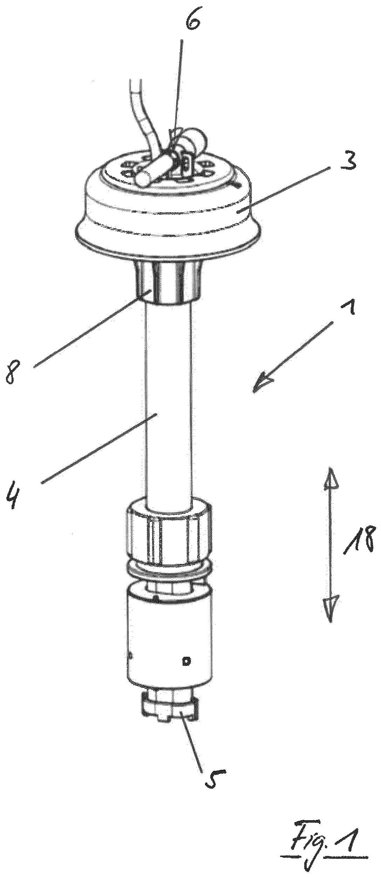

Fig. 1 lässt in schematisch perspektivischer Darstellung eine Sauglanze 1 nach der Offenbarung erkennen.

Die Sauglanze 1 verfügt über ein Saugrohr 4. Auf diesem Saugrohr 4 ist in Längsrichtung 18 des Saugrohrs 4 verschieblich ein Hülsenelement 8 gelagert. Das Hülsenelement 8 dient seinerseits der Anordnung eines kappenartig ausgebildeten Koppelelements 3. Dieses ist aus einem federelastischen Material gebildet, beispielsweise Silikon oder Gummi.

Das Hülsenelement 8 trägt oberseitig des Weiteren eine Stutzenvorrichtung 6, die im gezeigten Ausführungsbeispiel über zwei Stutzen 9 und 10 verfügt, die dem jeweiligen Anschluss einer Verschlauchung 22 dienen.

Das Saugrohr 4 trägt ein Fußteil 5, das im bestimmungsgemäßen Verwendungsfall dafür Sorge trägt, dass die fußseitig bereitgestellte Ansaugöffnung des Saugrohrs 4 beabstandet zum Boden eines Behälters 2 zu liegen kommt, so dass eine vom Behälter bevorratete Flüssigkeit fußseitig der Sauglanze 1 angesogen werden kann.

Aufgrund der in Längsrichtung 18 verschiebbaren Anordnung des Hülsenelements 8 am Saugrohr 4 ist auch das vom Hülsenelement 8 getragene Koppelelement 3 in Längsrichtung 18 verschiebbar am Saugrohr 4 angeordnet. Insofern ist unter Zwischenordnung des Hülsenelements 8 eine verschiebbare Anordnung des Koppelelements 3 am Saugrohr 4 gegeben. Das Hülsenelement 8 kann deshalb auch als Schlitten bezeichnet werden.

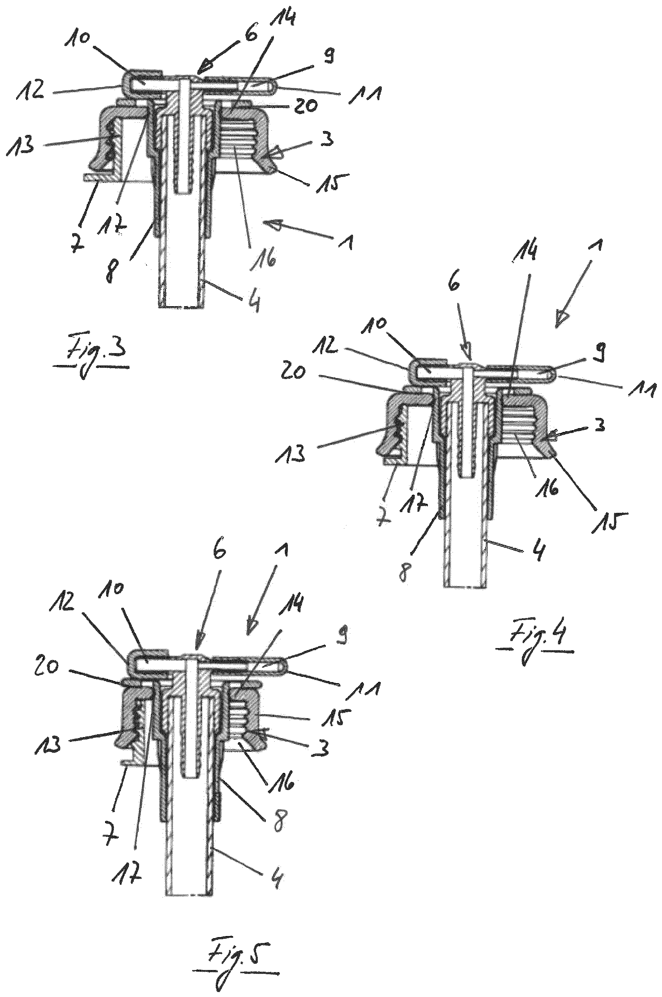

Das Koppelelement 3 dient im bestimmungsgemäßen endmontierten Zustand dem dichten Anliegen an einem Anschlussstutzen 13 eines zugehörigen Vorratsbehälters 2, wie sich dies aus einer Zusammenschau der Figuren 3 bis 7 ergibt.

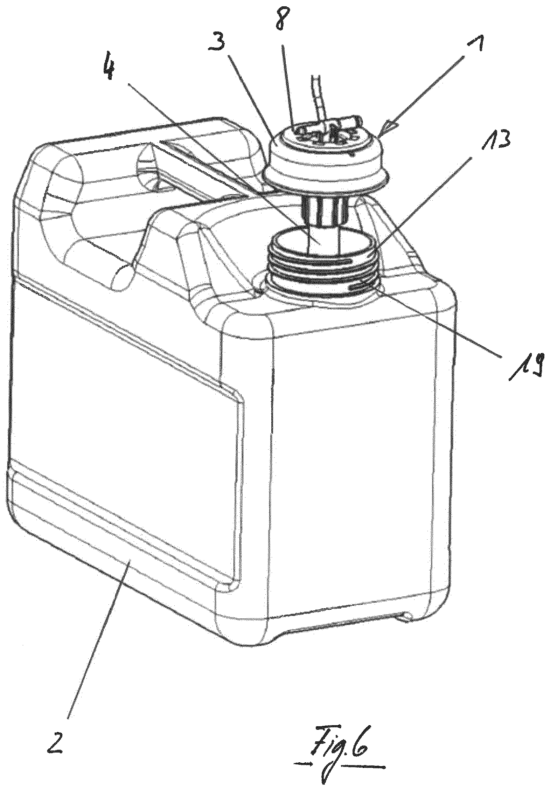

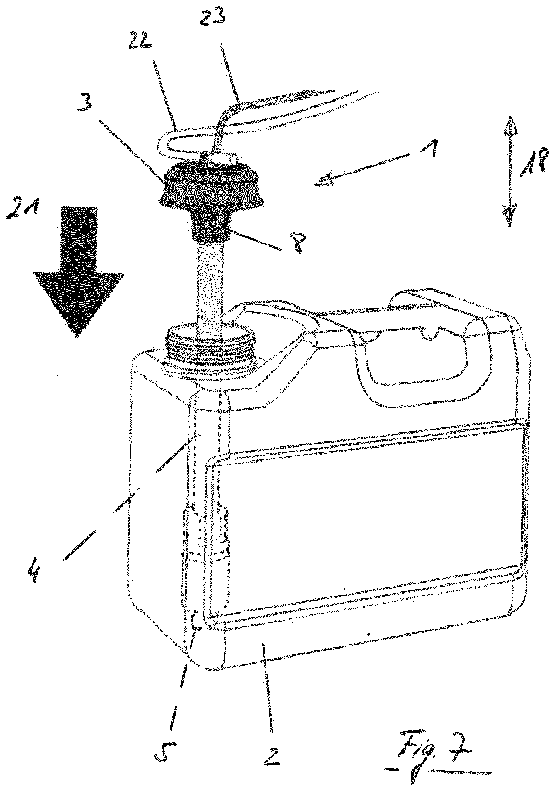

Die Figuren 6 und 7 lassen insbesondere die Montage der Sauglanze 1 erkennen.

Zur Anordnung der Sauglanze 1 im Vorratsbehälter 2 ist die Sauglanze 1 mit ihrem Fußteil 5 voran durch die vom Anschlussstutzen 13 des Vorratsbehälters 2 bereitgestellte Behälteröffnung zu führen, und zwar so weit, bis das Fußteil 5 auf den behälterseitigen Boden zu stehen kommt, wie dies insbesondere die Darstellung nach Fig. 7 erkennen lässt. Für eine lagesichere und positionsfeste Anordnung der Sauglanze 1 in Relation zum Vorratsbehälter 2 ist nun das Hülsenelement 8 zusammen mit dem davon getragenen Koppelelement 3 in Längsrichtung 18 verwenderseitig zu verfahren, und zwar mit Bezug auf die Zeichnungsebene nach Fig. 7 nach unten in Richtung des Pfeils 21. Infolge dieser Verfahrbewegung wird das kappenförmig ausgebildete Koppelelement 3 über den Anschlussstutzen 13 des Behälters 2 gestülpt.

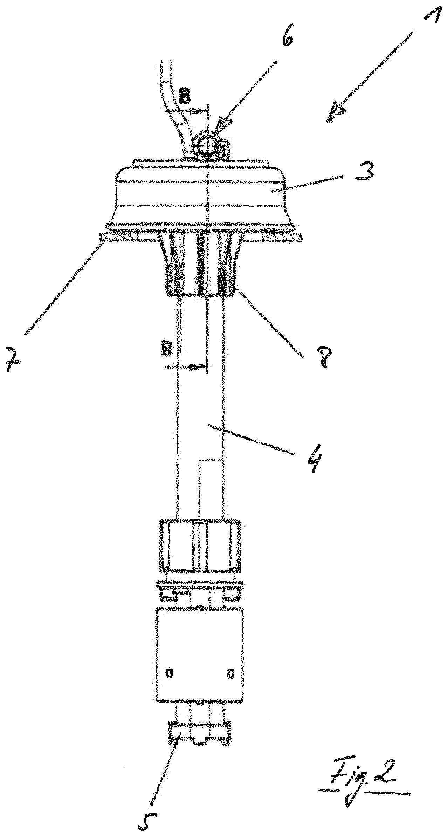

Die Anordnung des Koppelelements 3 am Anschlussstutzen 13 im endmontierten Zustand lässt insbesondere Fig. 3 erkennen, die einen Schnitt entlang der Schnittlinie B-B nach Fig. 2 zeigt.

Das Koppelelement 3 ist kappenförmig ausgebildet und verfügt über einen Kappendeckel 14 sowie über eine Wandung 15. Dabei sind der Kappendeckel 14 und die Wandung 17 einstückig ausgebildet und bestehen aus einem elastischen Material.

Die umlaufende Wandung 15 umgreift im endmontierten Zustand den Anschlussstutzen 13, wobei die Wandung auf ihrer dem Anschlussstutzen 13 im endmontierten Zustand zugewandten Innenoberfläche mit einer Haltekontur 16 ausgerüstet ist. Bei dieser Haltekontur 16 handelt es sich beispielsweise um Rastvorsprünge, die im endmontierten Zustand in das vom Anschlussstutzen 13 bereitgestellte Außengewinde 19 eingreifen. Wie sich aus der Darstellung nach Fig. 3 ferner ergibt, verfügt der Kappendeckel 14 über eine Durchtrittsöffnung 17, durch die hindurch das Hülsenelement 8 geführt ist. Das Hülsenelement 8 trägt seinerseits außenumfangsseitig eine umlaufende Rille 20, in die der Kappendeckel 14 mit seiner durch die Durchtrittsöffnung 17 gegebenen Randkante formschlüssig eingreift.

Das Koppelelement 3 nach der Offenbarung ist zu einer Mehrzahl von in der Größe und/oder Form unterschiedlichen Anschlussstutzen 13 größen- und/oder formkompatibel ausgebildet. Dies erbringt den Vorteil, dass das Koppelelement 3 in Kombination mit unterschiedlich ausgestalteten Anschlussstutzen 13 gleichermaßen bestimmungsgemäß verwendbar ist. Die Figuren 3, 4 und 5 lassen jeweils unterschiedlich groß ausgebildete Koppelelemente 3 erkennen, die jeweils zu einer Mehrzahl von jeweils unterschiedlich ausgebildeten Anschlussstutzen kompatibel sind.

Für eine bestimmungsgemäße Anordnung des Koppelelements 3 auf einem Anschlussstutzen 13 ist dieses verwenderseitig aufzudehnen, über den Anschlussstutzen 13 zu führen und alsdann wieder loszulassen, infolgedessen sich das Koppelelement 3 federelastisch zusammenzieht und sich dicht an die Außenoberfläche des Anschlussstutzens 13 anlegt.

Das Hülsenelement 8 ist mit einer Stutzeneinrichtung 6 zur Anordnung einer Verschlauchung 22 ausgerüstet. Dabei verfügt die Stutzeneinrichtung 6 im gezeigten Ausführungsbeispiel über zwei Stutzen 9 und 10, die wahlweise zur Anordnung der Verschlauchung 22 dienen. Dabei unterscheiden sich die Stutzen 9 und 10 hinsichtlich ihres freien Durchströmungsquerschnitts, wobei der Stutzen 9 im Unterschied zum Stutzen 10 im Durchmesser kleiner ausgebildet ist. Gemäß der Darstellung nach den Figuren 3 bis 5 sind die Stutzen 9 und 10 jeweils mit einer Kappe 11 beziehungsweise 12 verschlossen. Im endmontierten Zustand ist eine dieser beiden Kappen 11 beziehungsweise 12 entfernt, so dass der jeweils zugehörige Stutzen 9 beziehungsweise 10 dem Anschluss einer Verschlauchung 22 dient.

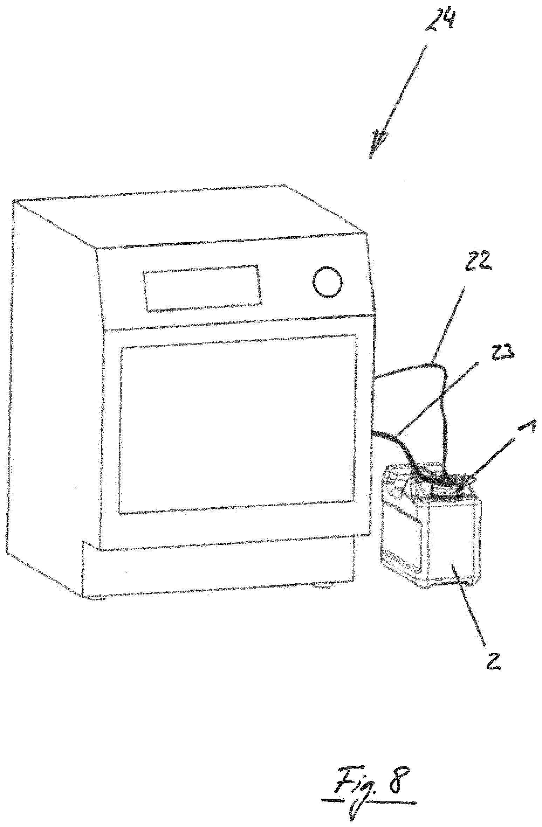

Fig. 8 zeigt ein Reinigungs- und/oder Desinfektionsgerät 24, dessen Behälter 2 mit einer offenbarungsgemäßen Sauglanze 1 ausgerüstet ist. Dabei steht die Sauglanze 1 in Wirkverbindung mit dem Reinigungs- und/oder Desinfektionsgerät 24 über die Verschlauchung 22 und eine Signal- und/oder Stromleitung 23.

### Bezugszeichen

- 1: Sauglanze
- 2: Behälter
- 3: Koppelelement
- 4: Saugrohr
- 5: Fußteil
- 6: Stutzeneinrichtung
- 7: Behälterwand
- 8: Hülsenelement
- 9: Stutzen
- 10: Stutzen
- 11: Kappe
- 12: Kappe
- 13: Anschlussstutzen
- 14: Kappendeckel
- 15: Wandung
- 16: Haltekontur
- 17: Durchtrittsöffnung
- 18: Längsrichtung
- 19: Außengewinde
- 20: Rille
- 21: Pfeil
- 22: Verschlauchung
- 23: Signal- und/oder Stromleitung
- 24: Reinigungs- und/oder Desinfektionsgerät

## Patentansprüche

1. Sauglanze für die Entnahme von Prozesschemikalien aus einem Kanister eines Reinigungs- und/oder Desinfektionsgeräts mit einem Saugrohr (4) und einem Koppelelement (3), wobei das Koppelelement (3) der lösbaren Anordnung des Saugrohrs (4) an einem kanisterseitigen Anschlussstutzen (13) dient,
und wobei das Koppelelement (3) nach Art einer Kappe ausgebildet ist und einen Kappendeckel (14) sowie eine umlaufende Wandung (15) aufweist, wobei die Wandung (15) den Anschlussstutzen (13) im endmontierten Zustand umgreifend ausgebildet ist, und wobei der Kappendeckel (14) eine Durchtrittsöffnung (17) für das Saugrohr (4) aufweist,
**dadurch gekennzeichnet,**
**dass** das Koppelelement (3) zumindest abschnittsweise aus einem elastisch verformbaren Material gebildet und derart ausgebildet ist, dass es verwenderseitig zur Anpassung an einen kanisterseitigen Anschlussstutzen (13) größen- und/oder formgedehnt werden kann und zu einer Mehrzahl von in der Größe und/oder Form unterschiedlichen Anschlussstutzen (13) größen- und/oder formkompatibel ausgebildet ist,
und **dass** die Sauglanze außerdem ein Hülsenelement (8) aufweist, das in Längsrichtung des Saugrohrs (4) verschiebbar auf dem Saugrohr (4) angebracht ist, und welches auf seiner dem Saugrohr (4) im endmontierten Zustand abgewandten Außenoberfläche eine Befestigungskontur zur Anordnung des Koppelelements (3) an dem Hülsenelement (8) aufweist,
so dass unter Zwischenordnung des Hülsenelements (8) eine verschiebbare Anordnung des Koppelelements (3) am Saugrohr (4) gegeben ist.

2. Sauglanze nach Anspruch 1, **dadurch gekennzeichnet, dass** das elastisch verformbare Material Silikon oder Gummi ist.

3. Sauglanze nach Anspruch 1 oder 2, **dadurch gekennzeichnet, dass** die Wandung (15) auf ihrer dem Anschlussstutzen (13) im endmontierten Zustand zugewandten Innenoberfläche mit einer Haltekontur (16) ausgerüstet ist.

4. Sauglanze nach einem der vorhergehenden Ansprüche, **dadurch gekennzeichnet, dass** die Befestigungskontur eine hülsenelementaußenseitig umlaufende Rille (20) ist.

5. Sauglanze nach einem der vorhergehenden Ansprüche, **dadurch gekennzeichnet, dass** das Koppelelement (3) am Saugrohr (4) und/oder am Hülsenelement (8) auswechselbar angeordnet ist.

6. Reinigungs- und/oder Desinfektionsgerät mit einer Sauglanze nach einem der vorhergehenden Ansprüche 1 bis 5.

## Claims

1. Suction lance for extracting process chemicals from a canister of a cleaning and/or disinfection device having a suction pipe (4) and a coupling element (3), the coupling element (3) serving the detachable arrangement of the suction pipe (4) on a canister-side connection nozzle (13),
and the coupling element (3) being designed in the form of a cap and having a cap cover (14) and a surrounding wall (15), the wall (15) being designed to encompass the connecting nozzle (13) in the fully assembled state, and the cap cover (14) having a passage opening (17) for the suction pipe (4),
**characterised**
**in that** the coupling element (3) is formed, at least in some portions, from an elastically deformable material and is designed in such a way that it can be stretched in size and/or shape by the user to adapt to a canister-side connection nozzle (13) and is designed to be compatible in size and/or shape with a plurality of connection nozzles (13) that differ in size and/or shape,
and **in that** the suction lance also has a sleeve element (8) which is movably mounted on the suction pipe (4) in the longitudinal direction of the suction pipe (4) and has, on its outer surface which faces away from the suction pipe (4) in the final assembled state, a fastening contour for arranging the coupling element (3) on the sleeve element (8)
so that, with intermediate arrangement of the sleeve element (8), a movable arrangement of the coupling element (3) on the suction pipe (4) is present.

2. Suction lance according to claim 1, **characterised in that** the elastically deformable material is silicone or rubber.

3. Suction lance according to claim 1 or 2, **characterised in that** the wall (15) is equipped with a retaining contour (16) on its inner surface which faces the connection nozzle (13) in the final assembled state.

4. Suction lance according to any of the preceding claims,
**characterised in that** the fastening contour is a groove (20) which surrounds the sleeve element on the outside.

5. Suction lance according to any of the preceding claims,
**characterised in that** the coupling element (3) is interchangeably arranged on the suction pipe (4) and/or on the sleeve element (8).

6. Cleaning and/or disinfection device having a suction lance according to any of the preceding claims 1 to 5.

## Revendications

1. Lance d'aspiration destinée au prélèvement de produits chimiques de traitement à partir d'un bidon d'un appareil de nettoyage et/ou de désinfection comportant un tube d'aspiration (4) et un élément d'accouplement (3), dans laquelle l'élément d'accouplement (3) sert à l'agencement amovible du tube d'aspiration (4) sur un raccord (13) côté bidon,
et dans laquelle l'élément d'accouplement (3) est conçu à la manière d'un capuchon et présente un couvercle (14) ainsi qu'une paroi (15) périphérique, dans laquelle la paroi (15) est conçue de manière à entourer le raccord (13) à l'état monté définitif, et dans laquelle le couvercle (14) présente une ouverture de passage (17) pour le tube d'aspiration (4),
**caractérisée en ce**
**que** l'élément d'accouplement (3) est constitué, au moins dans certaines sections, d'un matériau élastiquement déformable et est conçu de telle sorte que, du côté utilisateur, il puisse être étiré en taille et/ou en forme pour épouser un raccord (13) côté bidon et qu'il soit compatible, en termes de taille et/ou de forme, avec une pluralité de raccords (13) de tailles et/ou de formes différentes,
et **en ce que** la lance d'aspiration présente en outre un élément manchon (8) qui est monté sur le tube d'aspiration (4) de manière à pouvoir coulisser dans le sens longitudinal du tube d'aspiration (4), et qui présente, sur sa surface extérieure opposée au tube d'aspiration (4) dans l'état monté définitif, un contour de fixation permettant d'agencer l'élément d'accouplement (3) sur l'élément manchon (8),
en sorte que, grâce à l'intercalation de l'élément manchon (8), l'élément d'accouplement (3) puisse être agencé de manière coulissante sur le tube d'aspiration (4).

2. Lance d'aspiration selon la revendication 1, **caractérisée en ce que** le matériau élastiquement déformable est du silicone ou du caoutchouc.

3. Lance d'aspiration selon la revendication 1 ou 2, **caractérisée en ce que** la paroi (15) est pourvue, sur sa surface intérieure tournée vers le raccord (13) à l'état monté définitif, d'un contour de retenue (16).

4. Lance d'aspiration selon l'une des revendications précédentes, **caractérisée en ce que** le contour de fixation est une rainure (20) s'étendant sur tout le pourtour extérieur de l'élément manchon.

5. Lance d'aspiration selon l'une des revendications précédentes, **caractérisée en ce que** l'élément d'accouplement (3) est agencé de manière interchangeable sur le tube d'aspiration (4) et/ou sur l'élément manchon (8).

6. Appareil de nettoyage et/ou de désinfection comportant une lance d'aspiration selon l'une des revendications 1 à 5 précédentes.
